# EUROPEAN PATENT APPLICATION

(11) **EP 4 744 630 A1**
(43) Date of publication of application: **20.05.2026**
(21) Application number: 24861493.5
(22) Date of filing: 12.04.2024
(51) Int. Cl.: A61F 5/00, A61F 2/04, A61L 29/06, A61L 29/14

(54) **EXTENDABLE DIGESTIVE TRACT CANNULA AND USE THEREOF**

(30) Priority: 08.09.2023 CN 202311159684
(71) Applicant: Hangzhou Tangji Medical Technology Co., Ltd., Hangzhou, Zhejiang 310052 (CN)
(72) Inventor: LI, Wenyu, Hangzhou, Zhejiang 310052 (CN)
(74) Representative: Vogelbruch, Keang
(86) International application number: PCT/CN2024/087412
(87) International publication number: WO 2025/050643

(57) **Abstract**

An extendable digestive tract cannula (100) and a use thereof, relating to the technical field of medical devices. The extendable digestive tract cannula (100) comprises a first cannula (110), a second cannula (120) and a degrading material (130). The first cannula (110) has two opposite ends, namely a proximal end and a distal end, the first cannula (110) and the second cannula (120) are sleeved, and a double-layer structure is formed at the distal end of the first cannula (110). The double-layer structure is fixed by means of the degrading material (130). When the degrading material (130) is degraded, the second cannula (120) moves toward an end surface of the distal end of the first cannula (110). By means of providing the movable second cannula (120), after the degrading material (130) is degraded, the second cannula (120) moves backward, thereby achieving the purpose of extending the digestive tract cannula (100). As the length of the digestive tract cannula (100) increases, the area preventing intestinal absorption also increases, which weakens the intestinal compensatory absorption function at a distal end of the digestive tract cannula (100), improves the isolation effect between chyme and the intestine, and can reduce the digestion and absorption effect of the intestine for a relatively long time.

## Description

### CROSS REFERENCE TO RELATED APPLICATION

The present invention claims the priority to the Chinese patent application with the filing No. 2023111596845 filed with the Chinese Patent Office on September 8, 2023, and entitled "EXTENDABLE DIGESTIVE TRACT CANNULA AND USE THEREOF", which is incorporated herein by reference in entirety.

### TECHNICAL FIELD

The present invention relates to the technical field of medical devices, and in particular to an extendable digestive tract cannula (sleeve) and use thereof.

### BACKGROUND ART

With the improvement of living standards, the incidence of obesity is also increasing year by year. Currently, global annual expenditure related to obesity amounts to approximately 2.8% of the global GDP, which confirms the fact that the number of obese individuals is increasing annually. According to statistics, about 80% to 90% of obese individuals have type II diabetes, which is the most common type of diabetes, accounting for about 90% of all diabetes cases worldwide. Currently, diabetes has become the third major non-communicable disease after cardiovascular diseases and cancer. The number of patients is increasing annually, making it a global public health problem threatening human health.

Currently, the pathogenesis of type II diabetes is still not entirely clear. Despite existing oral medications and insulin therapy, some patients still have poor blood glucose control, and cases of complications still occur and progress. Bariatric surgery has good therapeutic effects on the aforementioned diseases, but it involves irreversible physiological trauma changes, a certain mortality rate, and postoperative complications, such as digestive tract leakage, anastomotic stenosis, or dumping syndrome. Currently, a duodenal-jejunal bypass sleeve (DJBS) has been developed based on the principle of gastric bypass surgery. By placing the sleeve in the duodenum and proximal jejunum, contact between chyme and the intestinal wall is isolated. After ingested nutrients enter the stomach, they pass through the sleeve into the proximal jejunum; pancreatic juice and bile are naturally secreted, flow downward between the sleeve and the intestinal wall, and mix with the chyme in the distal end (i.e., the jejunum) of the duodenal-jejunal bypass sleeve (DJBS), thereby reducing absorption and serving the purpose of treating obesity and type II diabetes.

However, as research progresses, it has been found that while the digestive tract sleeve can reduce intestinal absorption of food in the short term after implantation, intestinal absorption of food increases again over time, failing to achieve a more lasting reduction in intestinal digestion and absorption.

In view of this, the present invention is specifically proposed.

### SUMMARY

An objective of the present invention is to provide an extendable digestive tract sleeve and use thereof, which can achieve controllable extension of the digestive tract sleeve, weaken the function of intestinal compensatory absorption at the distal end of the digestive tract sleeve, and improve the isolation between chyme and the intestinal tract.

The embodiments of the present invention are implemented as follows.

In a first aspect, the present invention provides an extendable digestive tract sleeve, including a first sleeve, a second sleeve, and a degradable material; opposite ends of the first sleeve are a proximal end and a distal end, respectively; the first sleeve is sleeved with the second sleeve, and a double-layer structure is formed at the distal end of the first sleeve; the double-layer structure is fixed by the degradable material; and after the degradable material degrades, the second sleeve moves toward the end face of the distal end of the first sleeve.

In an optional embodiment, the degradation time of the degradable material is at least 30 days.

Preferably, the degradation time of the degradable material is 60 days to 90 days.

Preferably, the bonding force between the degradable material and the first sleeve is at least 2.5 N; more preferably, the bonding force between the degradable material and the first sleeve is at least 10 N.

Preferably, the degradable material is an annular diaphragm having a thickness of 0.01 mm to 0.06 mm.

In an optional embodiment, the degradable material includes at least one selected from polylactide and polyglycolide.

Preferably, the degradable material includes one or more selected from the group consisting of poly(DL-lactide-co-glycolide), polyglycolide-polycaprolactone, poly(L-lactide-co-ε-caprolactone), poly(L-lactic acid), and poly(L-lactic acid)-trimethyl chitosan.

Preferably, in the poly(DL-lactide-co-glycolide), a mass ratio of DL-lactide to glycolide is 0.8-1.2:0.8-1.2; in the polyglycolide-polycaprolactone, a mass ratio of polyglycolide to polycaprolactone is 7-8:2-3; in the poly(L-lactide-co-ε-caprolactone), a mass ratio of L-lactide to ε-caprolactone is 6.5-7.5:2.5-3.5; and in the poly(L-lactic acid)-trimethyl chitosan, a mass ratio of poly(L-lactic acid) to trimethyl chitosan is 6.5-7.5:2.5-3.5.

In an optional embodiment, the degradable material is connected to the first sleeve by calendering or RF welding.

In an optional embodiment, the first sleeve and the second sleeve are formed by folding a single tube or welding two tubes, and the welding point for welding the two tubes is the distal end of the first sleeve.

Preferably, the second sleeve is located inside the first sleeve.

In an optional embodiment, after the degradable material degrades, the second sleeve extends out of the first sleeve by a length of 10 cm to 30 cm.

In an optional embodiment, the length of the first sleeve is at least 30 cm, and the length of the second sleeve is 15 cm to 80 cm.

In an optional embodiment, each of the first sleeve and the second sleeve has a thickness of 0.01 mm to 0.03 mm.

In an optional embodiment, the materials of the first sleeve and the second sleeve include at least one selected from the group consisting of polytetrafluoroethylene, fluorinated ethylene propylene copolymer, expanded polytetrafluoroethylene, polyurethane, low-density polyethylene, and linear low-density polyethylene.

In a second aspect, the present invention provides a use of the extendable digestive tract sleeve according to any one of the aforementioned embodiments in a product for reducing digestive tract absorption.

The beneficial effects of the embodiments of the present invention are:

The present invention provides an extendable digestive tract sleeve and use thereof. By providing a movable second sleeve, after the degradable material degrades, the second sleeve moves backward, thereby achieving the objective of extending the digestive tract sleeve. As the length of the digestive tract sleeve increases, the area avoiding intestinal absorption also increases, the function of intestinal compensatory absorption at the distal end of the digestive tract sleeve is weakened, the isolation between chyme and the intestinal tract is improved, and the effect of intestinal digestion and absorption can be reduced for a relatively long duration.

### BRIEF DESCRIPTION OF DRAWINGS

To describe the technical solutions in the embodiments of the present invention more clearly, the following briefly introduces the drawings required for describing the embodiments. It should be understood that the following drawings show only some embodiments of the present invention and therefore should not be considered as limiting the scope. For those of ordinary skill in the art, other related drawings can be obtained from these drawings without inventive effort.
FIG. 1 is a schematic structural diagram of an extendable digestive tract sleeve provided by an embodiment of the present invention; and
FIG. 2 is a schematic structural diagram of the extendable digestive tract sleeve provided by an embodiment of the present invention after extension.

Reference numerals: 100, extendable digestive tract sleeve; 110, first sleeve; 120, second sleeve; 130, degradable material.

### DETAILED DESCRIPTION OF EMBODIMENTS

The embodiments of the present invention will be described in detail below with reference to examples, but those skilled in the art will understand that the following examples are only used to illustrate the present invention and should not be construed as limiting the scope of the present invention. Where specific conditions are not indicated in the examples, they are carried out under conventional conditions or conditions recommended by the manufacturer. Reagents or instruments for which the manufacturer is not specified are conventional products commercially available.

The endpoints of ranges and any values disclosed in the present invention are not limited to the precise ranges or values, and such ranges or values should be understood to include values close to these ranges or values. For numerical ranges, the endpoint values of the ranges, the endpoint values and individual point values, and individual point values may be combined with each other to obtain one or more new numerical ranges, which should be considered as specifically disclosed herein.

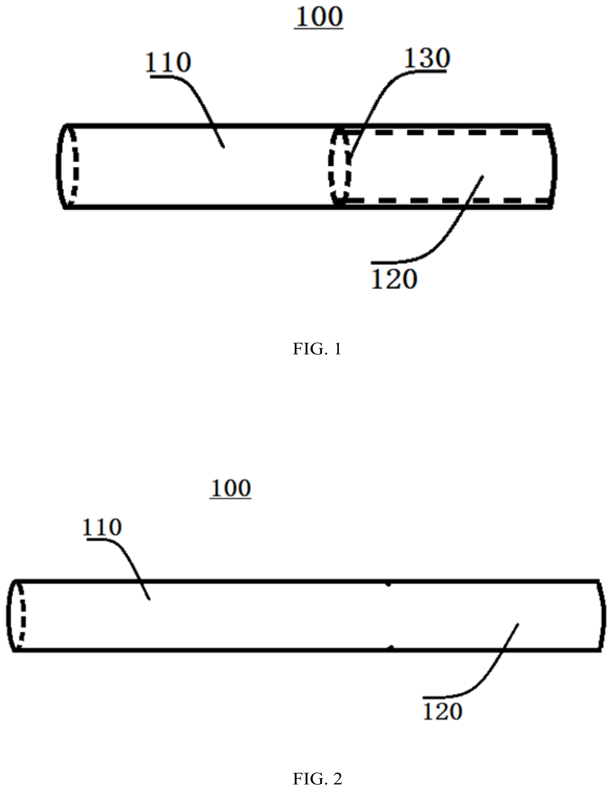

Referring to FIG. 1 and FIG. 2, the present invention provides an extendable digestive tract sleeve 100, including a first sleeve 110, a second sleeve 120, and a degradable material 130. Opposite ends of the first sleeve 110 are a proximal end and a distal end, respectively. The first sleeve 110 is sleeved with the second sleeve 120, and a double-layer structure is formed at the distal end of the first sleeve 110. The double-layer structure is fixed by the degradable material 130. The degradable material 130 has degradability after the digestive tract sleeve is used for a period of time. After the degradable material 130 degrades, the second sleeve 120 moves toward the end face of the distal end of the first sleeve 110.

Currently, the method of implanting a digestive tract sleeve can reduce the absorption of food by the intestinal wall, achieving effects such as weight loss or blood glucose reduction. However, as the implantation time of the digestive tract sleeve increases, the jejunum at the distal end of the sleeve gradually develops compensatory changes, such as lengthening of intestinal mucosal villi, increased folds, deepening of intestinal gland crypts, thickening or lengthening of the intestinal tube, and thickening of the intestinal wall. The occurrence of these compensatory phenomena increases the intestinal absorption capacity for food, thereby affecting changes in weight and metabolism. Therefore, the inventor inventively proposes an extendable digestive tract sleeve. By providing the second sleeve 120, after the degradable material 130 degrades, the second sleeve 120 moves away from the stomach along with intestinal peristalsis, achieving the objective of extending the digestive tract sleeve. The lengthening of the digestive tract sleeve weakens the function of intestinal compensatory absorption at its distal end, improves the isolation between chyme and the intestine, and further reduces intestinal digestion and absorption of food.

In an optional embodiment, ideally, the second sleeve 120 should extend after the first sleeve 110 initially reduces the digestion and absorption effect, i.e., when the intestinal wall at the distal end of the originally implanted digestive tract sleeve begins to exhibit compensatory absorption, thereby achieving the effect of reducing digestion and absorption again. Therefore, it is necessary to control the degradation time of the degradable material 130 to be at least 30 days to ensure that the first sleeve 110 fully functions.

Preferably, to ensure that the first sleeve 110 can fully exert its effect of reducing intestinal digestion and absorption, the degradation time of the degradable material 130 is 60 days to 90 days.

Preferably, to ensure that the degradable material 130 degrades according to the aforementioned time, allowing the second sleeve 120 to extend smoothly, it is necessary to ensure that the bonding force between the degradable material 130 and the first sleeve 110 is at least 2.5 N; more preferably, the bonding force between the degradable material 130 and the first sleeve 110 is at least 10 N.

Preferably, the degradable material 130 is an annular diaphragm. To ensure that the degradable material 130 degrades according to the aforementioned time, allowing the second sleeve 120 to extend smoothly, the annular diaphragm has a thickness of 0.01 mm to 0.06 mm.

In an optional embodiment, to ensure that the second sleeve 120 can extend out of the first sleeve 110 within a fixed time, the selection of the degradable material 130 is one of the key influencing factors.

Preferably, the degradable material 130 includes at least one selected from polylactide and polyglycolide.

Preferably, the degradable material 130 includes one or more selected from the group consisting of the group consisting of poly(DL-lactide-co-glycolide), polyglycolide-polycaprolactone, poly(L-lactide-co-ε-caprolactone), poly(L-lactic acid), and poly(L-lactic acid)-trimethyl chitosan.

Preferably, to ensure the degradation characteristics of the degradable material 130, it is necessary to control the composition ratio of the aforementioned composite degradable material 130. For example, in poly(DL-lactide-co-glycolide), a mass ratio of DL-lactide to glycolide is 0.8-1.2:0.8-1.2; in polyglycolide-polycaprolactone, a mass ratio of polyglycolide to polycaprolactone is 7-8:2-3; in poly(L-lactide-co-ε-caprolactone), a mass ratio of L-lactide to ε-caprolactone is 6.5-7.5:2.5-3.5; and in poly(L-lactic acid)-trimethyl chitosan, a mass ratio of poly(L-lactic acid) to trimethyl chitosan is 6.5-7.5:2.5-3.5. By controlling the composition ratio of the degradable material 130 within the above range, the prepared degradable material 130 can satisfy the bonding force requirement with the first sleeve 110.

In some embodiments, the performance of the degradable material 130 is related to the specific selection of its raw materials. Different raw material selections, or the same raw materials with different ratios, may lead to different degradation times for the degradable material 130. For example, when the degradation time of the degradable material 130 is about one month, poly(L-lactic acid)-trimethyl chitosan and poly(DL-lactide-co-glycolide) can be selected as raw materials in a mass ratio of 1-2:8-9; when the degradation time of the degradable material 130 is 1-2 months, poly(L-lactic acid)-trimethyl chitosan and poly(DL-lactide-co-glycolide) can be selected as raw materials in a mass ratio of 3:7; when the degradation time of the degradable material 130 is about 3 months, poly(L-lactic acid)-trimethyl chitosan and poly(DL-lactide-co-glycolide) can be selected as raw materials in a mass ratio of 1:1; and when the degradation time of the degradable material 130 is about 4 months, poly(L-lactide-co-ε-caprolactone) and poly(DL-lactide-co-glycolide) can be selected as raw materials in a mass ratio of 6-8:2-4, or poly(L-lactic acid)-trimethyl chitosan and poly(DL-lactide-co-glycolide) can be selected as raw materials in a mass ratio of 6-8:2-4.

In an optional embodiment, the preparation method of the degradable material 130 includes solvent casting or spinning.

In an optional embodiment, the degradable material 130 is connected to the first sleeve 110 by calendering or RF welding.

In an optional embodiment, the first sleeve 110 and the second sleeve 120 are formed by folding a single tube or welding two tubes, and the welding point for welding the two tubes is at the distal end of the first sleeve 110. When the first sleeve 110 and the second sleeve 120 are formed by folding a single tube, their diameters are the same; when the first sleeve 110 and the second sleeve 120 are formed by welding two tubes, their diameters differ slightly depending on their relative positions, with the diameter of the outer sleeve needing to be slightly larger than the diameter of the inner sleeve. Preferably, to simplify the manufacturing process, the first sleeve 110 and the second sleeve 120 have a structure formed by folding a single tube.

Since the second sleeve 120 needs to move toward the distal end of the first sleeve 110 after the degradable material 130 degrades, to facilitate better movement of the second sleeve 120, preferably, the second sleeve 120 is located inside the first sleeve 110. When the degradable material 130 fails, the second sleeve 120 can invert within the first sleeve 110 under the pushing action of chyme and move toward the distal end of the first sleeve 110 without being obstructed by the digestive tract wall.

Preferably, the manner in which the second sleeve 120 moves distally can be translation or inversion, more preferably inversion, and even more preferably, inversion into the lumen of the digestive tract sleeve.

In an optional embodiment, after the degradable material 130 degrades, the second sleeve 120 extends out of the first sleeve 110 by a length of 10 cm to 30 cm.

In an optional embodiment, the first sleeve 110 has a length of at least 30 cm, and the second sleeve 120 has a length of 15 cm to 80 cm.

In an optional embodiment, each of the first sleeve 110 and the second sleeve 120 has a thickness of 0.01 mm to 0.03 mm. The thicknesses of the first sleeve 110 and the second sleeve 120 can be the same or different, as long as they are both within the aforementioned range.

Preferably, to allow better inversion of the second sleeve 120, the length of the second sleeve 120 is less than the length of the first sleeve 110.

In an optional embodiment, the materials of the first sleeve 110 and the second sleeve 120 include at least one selected from the group consisting of polytetrafluoroethylene (PTFE), fluorinated ethylene propylene copolymer (FEP), expanded polytetrafluoroethylene (ePTFE), polyurethane (PU), low-density polyethylene (LDPE), and linear low-density polyethylene (LLDPE). The first sleeve 110 and the second sleeve 120 made of the aforementioned materials, in combination with the material of the degradable material 130, can ensure smooth extension of the second sleeve 120.

In a second aspect, the present invention provides a use of the extendable digestive tract sleeve 100 according to any one of the aforementioned embodiments in a product for reducing digestive tract absorption.

### Example 1

The example provides an extendable digestive tract sleeve 100, including a first sleeve 110, a second sleeve 120, and a degradable material 130. Opposite ends of the first sleeve 110 are a proximal end and a distal end, respectively. The first sleeve 110 and the second sleeve 120 are fixed by the degradable material 130, and a double-layer structure is formed at the distal end of the first sleeve 110. The degradable material 130 has degradability after the digestive tract sleeve is used for a period of time. After the degradable material 130 degrades, the second sleeve 120 moves toward the distal end.

In the example, the first sleeve 110 and the second sleeve 120 are formed by folding a single tube, and the second sleeve 120 is located inside the first sleeve 110. When the degradable material 130 fails, the second sleeve 120 inverts into the lumen of the digestive tract sleeve and moves toward the distal end of the first sleeve 110 under the pushing action of chyme, thereby completing the extension of the digestive tract sleeve. Therefore, the diameters of the first sleeve 110 and the second sleeve 120 are the same, both being 25 mm.

In this example, the material of the first sleeve 110 was low-density polyethylene, with a length of 30 cm and a thickness of 0.02 mm; the material of the second sleeve 120 was low-density polyethylene, with a length of 15 cm and a thickness of 0.02 mm.

In the example, the degradable material 130 was a mixture of poly(L-lactide-co-ε-caprolactone) and poly(DL-lactide-co-glycolide) mixed in a ratio of 8:2. Here, in the poly(L-lactide-co-ε-caprolactone), the mass ratio of lactide to caprolactone was 7:3; in the poly(DL-lactide-co-glycolide), the mass ratio of lactide to glycolide was 1:1.

The degradable material 130 was prepared by solution casting into an annular diaphragm, and the annular diaphragm was connected to the first sleeve 110 by laser welding. In this example, the annular diaphragm has a thickness of 0.03 mm.

### Example 2

The example provides an extendable digestive tract sleeve 100, the structure of which is the same as that in Example 1. The only differences are: a) The length of the first sleeve 110 was 40 cm; the length of the second sleeve 120 was 20 cm.
b) The proportion of the degradable material is different. In this example, in the degradable material, the mass ratio of poly(L-lactide-co-ε-caprolactone) to poly(DL-lactide-co-glycolide) was 1:1.

### Example 3

This example provides an extendable digestive tract sleeve 100, including a first sleeve 110, a second sleeve 120, and a degradable material 130. Opposite ends of the first sleeve 110 are a proximal end and a distal end, respectively. The first sleeve 110 and the second sleeve 120 are fixed by the degradable material 130, and a double-layer structure is formed at the distal end of the first sleeve 110. The degradable material 130 has degradability after the digestive tract sleeve is used for a period of time. After the degradable material 130 degrades, the second sleeve 120 moves toward the distal end.

In this example, the first sleeve 110 and the second sleeve 120 are formed by welding two tubes. The welding point for welding the two tubes is at the distal end of the first sleeve 110, and the second sleeve 120 is located inside the first sleeve 110. When the degradable material 130 fails, the second sleeve 120 inverts into the digestive tract sleeve and extends toward the distal end of the first sleeve 110 driven by chyme. Since the welding point fixes the first sleeve 110 and the second sleeve 120, the second sleeve 120 will not detach from the first sleeve 110 after extension.

Here, each of the first sleeve and the second sleeve had a thickness of 0.01 mm to 0.03 mm. Since the second sleeve 120 is sleeved on the surface of the first sleeve 110, the diameter of the second sleeve 120 needs to be larger than that of the first sleeve 110. To ensure smooth extension of the second sleeve 120 without extending out of the first sleeve 110, the diameter of the second sleeve 120 is larger than that of the first sleeve 110.

In this example, the material of the first sleeve 110 was an expanded polytetrafluoroethylene/perfluoroethylene-propylene copolymer composite, with a length of 50 cm, a thickness of 0.013 mm, and a diameter of 23 mm; the material of the second sleeve 120 was an expanded polytetrafluoroethylene/perfluoroethylene-propylene copolymer composite, with a length of 25 cm, a thickness of 0.013 mm, and a diameter of 25 mm.

In this example, the degradable material 130 was poly(L-lactic acid)-trimethyl chitosan, wherein the mass ratio of poly(L-lactic acid) to trimethyl chitosan was 7:3.

The degradable material 130 was prepared by solution casting and connected to the first sleeve 110 by RF welding.

### Example 4

This example provides an extendable digestive tract sleeve 100, the structure of which is the same as that in Example 3. The only differences are: a) The length of the first sleeve 110 was 40 cm; the length of the second sleeve 120 was 20 cm; the diameters of both the first sleeve 110 and the second sleeve 120 were 25 mm, and their thicknesses were both 0.03 mm. The materials of the first sleeve 110 and the second sleeve 120 were the same, both being low-density polyethylene.
b) The degradable material is different. The degradable material in this example was a mixture of poly(L-lactic acid)-trimethyl chitosan and poly(DL-lactide-co-glycolide), with a mixing ratio of 1:1.

### Example 5

This example provides an extendable digestive tract sleeve 100, the structure of which is the same as that in Example 1. The only differences are: a) The length of the first sleeve 110 was 60 cm; the length of the second sleeve 120 was 20 cm, and the diameter of the second sleeve 120 was 28 mm. The materials of both the first sleeve 110 and the second sleeve 120 were linear low-density polyethylene, with a thickness of 0.05 mm.
b) The proportion of the degradable material is different. In this example, in the degradable material, the mass ratio of poly(L-lactide-co-ε-caprolactone) to poly(DL-lactide-co-glycolide) was 7:3, and the annular diaphragm serving as the degradable material had a thickness of 0.06 mm.

### Example 6

This example provides an extendable digestive tract sleeve 100, the structure of which is the same as that in Example 3. The only differences are: a) The length of the first sleeve 110 was 30 cm; the length of the second sleeve 120 was 30 cm; the diameters of both the first sleeve 110 and the second sleeve 120 were 25 mm, and their thicknesses were both 0.03 mm. The materials of the first sleeve 110 and the second sleeve 120 were the same, both being polyurethane.
b) The degradable material is different. The degradable material in this example was a mixture of poly(L-lactic acid)-trimethyl chitosan and poly(DL-lactide-co-glycolide), with a mixing ratio of 2:8, and the annular diaphragm serving as the degradable material had a thickness of 0.04 mm.

### Example 7

This example provides an extendable digestive tract sleeve 100, the structure of which is the same as that in Example 1. The only differences are: a) The length of the first sleeve 110 was 70 cm, and the diameter of the first sleeve 110 was 20 mm; the length of the second sleeve 120 was 30 cm, and the diameter of the second sleeve 120 was 23 mm. The materials of both the first sleeve 110 and the second sleeve 120 were a mixture of linear low-density polyethylene and low-density polyethylene in a mass ratio of 1:1, with a thickness of 0.015 mm.
b) The proportion of the degradable material is different. The degradable material in this example was a mixture of poly(L-lactic acid)-trimethyl chitosan and poly(DL-lactide-co-glycolide), wherein the mass ratio of poly(L-lactic acid)-trimethyl chitosan to poly(DL-lactide-co-glycolide) was 3:7, and the annular diaphragm serving as the degradable material had a thickness of 0.05 mm.

### Example 8

This example provides an extendable digestive tract sleeve 100, the structure of which is the same as that in Example 1. The only differences are: a) The length of the first sleeve 110 was 90 cm, and the diameter of the first sleeve 110 is 20 mm; the length of the second sleeve 120 was 30 cm, and the diameter of the second sleeve 120 was 23 mm. The materials of both the first sleeve 110 and the second sleeve 120 were an expanded polytetrafluoroethylene/perfluoroethylene-propylene copolymer composite, with a thickness of 0.018 mm.
b) The degradable material was poly(p-dioxanone) (PDO), and the annular diaphragm serving as the degradable material had a thickness of 0.05 mm.

### Comparative Example 1

This comparative example provides a digestive tract sleeve, the structure of which is the same as that in Example 1. The only difference is that the connection between the second sleeve and the first sleeve did not involve a degradable material and was directly connected by laser welding.

### Comparative Example 2

This comparative example provides a digestive tract sleeve, the structure of which is the same as that in Example 2. The only difference is that the connection between the second sleeve and the first sleeve did not involve a degradable material and was directly connected by laser welding.

### Comparative Example 3

This comparative example provides a digestive tract sleeve, the structure of which is the same as that in Example 3. The only difference is that the connection between the second sleeve and the first sleeve did not involve a degradable material and was directly connected by RF welding.

### Comparative Example 4

This comparative example provides a digestive tract sleeve, the structure of which is the same as that in Example 4. The only difference is that the connection between the second sleeve and the first sleeve did not involve a degradable material and was directly connected by RF welding.

### Comparative Example 5

This comparative example provides a digestive tract sleeve, the structure of which is the same as that in Example 5. The only difference is that the connection between the second sleeve and the first sleeve did not involve a degradable material and was directly connected by laser welding.

### Comparative Example 6

This comparative example provides a digestive tract sleeve, the structure of which is the same as that in Example 6. The only difference is that the connection between the second sleeve and the first sleeve did not involve a degradable material and was directly connected by RF welding.

### Comparative Example 7

This comparative example provides a digestive tract sleeve, the structure of which is the same as that in Example 7. The only difference is that the connection between the second sleeve and the first sleeve did not involve a degradable material and was directly connected by laser welding.

### Comparative Example 8

This comparative example provides a digestive tract sleeve, the structure of which is the same as that in Example 8. The only difference is that the connection between the second sleeve and the first sleeve did not involve a degradable material and was directly connected by laser welding.

### Test Example 1

The extendable digestive tract sleeve 100 provided in Examples 1 to 8 and Comparative Examples 1 to 8 were tested. The test method was as follows:
1. The extendable digestive tract sleeve 100 prepared in Examples 1 to 8 and Comparative Examples 1 to 8 was completely immersed in simulated intestinal fluid (prepared according to the "Chinese Pharmacopoeia, 2022 Edition") and oscillated at 40 rpm in a shaker at 37 ± 2°C for different durations up to the set time. The sleeve was then gently removed from the simulated intestinal fluid, surface liquid was blotted with absorbent paper, one end of the first sleeve was fixed, the distal end of the second sleeve was cut, and the extendable part was fixed to the fixture of a computer-controlled material testing machine. The speed was set to 20 mm/min, and the initial bonding force was measured. If the force when the second sleeve separates from the first sleeve is less than 1 N, it is considered to have reached the degradation time.

The digestive tract sleeve after the degradation test was placed in a U-shaped pipeline testing device, and the extension of the second sleeve was observed under an instantaneous pressure difference of 30 cm H₂O. Here, the extension distance ratio = actual extension distance / theoretical extension distance. The results shown in Table 1 were obtained.

**Table 1 Disintegration of Degradable Materials**

| | Bonding Force Between Degradable Material and First Sleeve / N | Detachable Time Between Second Sleeve and First Sleeve / Days | Extension Distance Ratio of Second Sleeve at 30 cm H₂O |
|---|---|---|---|
| Example 1 | 12.98 | 120 | 98% |
| Example 2 | 18.92 | 100 | 98% |
| Example 3 | 23.95 | 178 | 100% |
| Example 4 | 22.82 | 101 | 100% |
| Example 5 | 20.31 | 130 | 100% |
| Example 6 | 22.82 | 32 | 100% |
| Example 7 | 19.27 | 47 | 100% |
| Example 8 | 18.32 | 180 | 100% |
| Comparative Example 1 | 36.5 | Does not detach | Unable to extend |
| Comparative Example 2 | 38.6 | Does not detach | Unable to extend |
| Comparative Example 3 | 38.90 | Does not detach | Unable to extend |
| Comparative Example 4 | 37.6 | Does not detach | Unable to extend |
| Comparative Example 5 | 85.78 | Does not detach | Unable to extend |
| Comparative Example 6 | 45.4 | Does not detach | Unable to extend |
| Comparative Example 7 | 28.65 | Does not detach | Unable to extend |
| Comparative Example 8 | 39.9 | Does not detach | Unable to extend |

| | | | |
|---|---|---|---|
| *Note: In Table 1, the bonding force between the degradable materials of Comparative Examples 1 to 8 and the first sleeve is the bonding force between the second sleeve and the first sleeve. | | | |

As can be seen from Table 1, the degradable material 130 of the extendable digestive tract sleeve 100 provided in the embodiments of the present invention has good disintegration ability, can be controlled to disintegrate after 30 days, with the longest disintegration time reaching 180 days, achieving an excellent extension effect for the digestive tract sleeve.

The above are only preferred embodiments of the present invention and are not intended to limit the present invention. For those skilled in the art, the present invention may have various modifications and variations. Any modifications, equivalent substitutions, or improvements, etc., made within the spirit and principles of the present invention shall be included within the protection scope of the present invention.

### INDUSTRIAL APPLICABILITY

The present invention can achieve controllable extension of the digestive tract sleeve, weaken the function of intestinal compensatory absorption at the distal end of the digestive tract sleeve, improve the isolation between chyme and the intestinal tract, and has good utility value.

## Claims

1. An extendable digestive tract sleeve, **characterized by** comprising a first sleeve, a second sleeve, and a degradable material, wherein opposite ends of the first sleeve are a proximal end and a distal end, respectively; the first sleeve is sleeved with the second sleeve, and a double-layer structure is formed at the distal end of the first sleeve; the double-layer structure is fixed by the degradable material; and after the degradable material degrades, the second sleeve moves toward the end face of the distal end of the first sleeve.

2. The extendable digestive tract sleeve according to claim 1, wherein a degradation time of the degradable material is at least 30 days;
preferably, the degradation time of the degradable material is 60 days to 180 days;
preferably, a bonding force between the degradable material and the first sleeve is at least 2.5 N; more preferably, the bonding force between the degradable material and the first sleeve is at least 10 N;
preferably, the degradable material is an annular diaphragm, and the annular diaphragm has a thickness of 0.01 mm to 0.06 mm.

3. The extendable digestive tract sleeve according to claim 2, wherein the degradable material comprises at least one selected from polylactide and polyglycolide;
preferably, the degradable material comprises one or more selected from a group consisting of poly(DL-lactide-co-glycolide), polyglycolide-polycaprolactone, poly(L-lactide-co-ε-caprolactone), poly(L-lactic acid), poly(L-lactic acid)-trimethyl chitosan, and poly(p-dioxanone);
preferably, in the poly(DL-lactide-co-glycolide), a mass ratio of DL-lactide to glycolide is 0.8-1.2:0.8-1.2; in the polyglycolide-polycaprolactone, a mass ratio of polyglycolide to polycaprolactone is 7-8:2-3; in the poly(L-lactide-co-ε-caprolactone), a mass ratio of L-lactide to ε-caprolactone is 6.5-7.5:2.5-3.5; and in the poly(L-lactic acid)-trimethyl chitosan, a mass ratio of poly(L-lactic acid) to trimethyl chitosan is 6.5-7.5:2.5-3.5.

4. The extendable digestive tract sleeve according to claim 1, wherein the degradable material is connected to the first sleeve by laser welding or RF welding.

5. The extendable digestive tract sleeve according to claim 1, wherein the first sleeve and the second sleeve are formed by folding a single tube or welding two tubes, and a welding point for welding the two tubes is at the distal end of the first sleeve;
preferably, the second sleeve is located inside the first sleeve.

6. The extendable digestive tract sleeve according to claim 1 or 5, wherein after the degradable material degrades, the second sleeve extends out of the first sleeve by a length of 10 cm to 30 cm.

7. The extendable digestive tract sleeve according to claim 6, wherein the first sleeve has a length of at least 30 cm, and the second sleeve has a length of 15 cm to 80 cm.

8. The extendable digestive tract sleeve according to claim 6, wherein each of the first sleeve and the second sleeve has a thickness of 0.01 mm to 0.03 mm.

9. The extendable digestive tract sleeve according to claim 6, wherein the materials of the first sleeve and the second sleeve comprise one or more composites selected from a group consisting of polytetrafluoroethylene, fluorinated ethylene propylene copolymer, expanded polytetrafluoroethylene, perfluoroethylene-propylene copolymer, polyurethane, low-density polyethylene, and linear low-density polyethylene.

10. A use of the extendable digestive tract sleeve according to any one of claims 1 to 9 in a product for reducing digestive tract absorption.
